# EUROPEAN PATENT APPLICATION

(11) **EP 4 140 530 A1**
(43) Date of publication of application: **01.03.2023**
(21) Application number: 21193778.4
(22) Date of filing: 30.08.2021
(51) Int. Cl.: A61M 39/02, A61M 5/42, A61M 25/02, A61M 5/158

(54) **STERILE VASCULAR ACCESS DEVICE**

(71) Applicant: MPG Medical AB, 181 63 Lidingö (SE)
(72) Inventor: Gellerfors, Mikael, 181 66 LIDINGÖ (SE); Gellerfors, Pär, 181 63 LIDINGÖ (SE)
(74) Representative: EIP

(57) **Abstract**

A sterile vascular access device for placing a central venous catheter (CVC) on a human subject, said device comprising:
a flexible enclosure formed of a flexible, transparent, pathogen impermeable medical grade material, said flexible enclosure having a base portion and a top portion, said base portion and said top portion together defining an internal space of the flexible enclosure,
said base portion having an adhesive bottom surface adapted to be reversibly attached to the skin of the subject and a top surface connected to the top portion, and
said top portion comprising a tubular portion having a proximal end which is connected to the top surface of the base portion and a distal end which is sealed.

## Description

### FIELD OF THE INVENTION

The present invention relates to a sterile vascular access device for placing a central venous catheter (CVC) on a human subject.

### BACKGROUND OF THE INVENTION

Patients in need of for example vasopressors, parenteral nutrition, dialysis, chemotherapy, long-term antibiotics, electrolytes, frequent blood draws, measuring central venous pressure, and/or patients with difficult peripheral vascular access, may need a central venous catheter (CVC).

Central venous catheters, often referred to as central lines, are commonly placed in the internal jugular vein, subclavian vein, axillary vein or femoral vein. Also, placement of peripherally inserted central catheters is possible. Central venous access is also needed to place pulmonary artery catheters and plasmapheresis and hemodialysis catheters, as well as to place inferior vena cava filters, introduce wires for transvenous pacing and defibrillator devices, and for venous interventions. Central venous access is a commonly performed procedure with approximately 8 percent of hospitalized patients requiring central venous access during the course of their hospital stay. More than five million central venous catheters are inserted in the United States each year.

Before insertion of a central venous catheter the provider must perform proper hygienic procedures, including washing hands, putting on surgical cap, sterile surgical gown and sterile gloves. Next, thorough cleaning of the needle and catheter insertion site is done, and surgical draping is applied to the patient. A local anesthetic is applied if necessary. The location of the vein is identified by landmarks or with the use of an ultrasound device. A hollow needle is then advanced through the skin until blood is aspirated. Using the Seldinger technique, a blunt guidewire is passed through the hollow needle after which the hollow needle is removed. A dilating device may be passed over the guidewire to expand the tract. Finally, the central venous catheter is then passed over the guidewire, which is then removed. All the lumens of the central venous catheter are flushed with saline. The central venous catheter is then sutured in place and covered by an occlusive dressing before the sterile draping of the patient is removed.

All vascular catheters can introduce bacteria into the bloodstream, but the risk is considerably higher with central venous catheters. Central venous catheters may cause sepsis from bacteria like for example *Staphylococcus aureus* and *Staphylococcus epidermidis.* In a 2011 UK national point prevalence study on healthcare associated infections and antimicrobial use, 40% of primary blood stream infections were related to a central venous catheter. An American case-control study of critically ill patients found that nosocomial blood stream infection was associated with increased mortality, length of stay in hospital and intensive care, and economic burden (Pittet D et al. JAMA1994; 271:1598-601). Central line-associated bloodstream infections have gained increasing attention in recent years. Proper hand washing, sterile surgical gown, sterile gloves, surgical cap, sterile draping over the patient and thorough cleaning of the needle and catheter insertion site are now routinely used to decrease the infection rate. In the US, The National Patient Safety Goals require documentation of a checklist for central venous catheters insertion. However, the hygienic measures are time consuming and otherwise challenging for among other the intensive care unit doctor on call. For example, other important time critical tasks cannot be performed during the period when the intensive care unit doctor is in sterile surgical gown and gloves. It is not unusual that the CVC procedure is interrupted and has to be continued at a later time. The preparations must then be repeated again.

In addition to central venous catheters as described above, there is a similar need for fast and sterile placement of dialysis catheters and femoral artery needles and/or catheters. Furthermore, fast and sterile methods may be needed during placement of other needles and/or catheters for venous access, arterial access, intraosseus access, regional- and/or neuroaxial anesthesia and peripherally inserted central catheters.

Accordingly, there is still a need for improved procedures which can alleviate the deficiencies of the existing procedures for sterile vascular access, e.g. placing a central venous catheter (CVC) on a patient.

### SUMMARY OF THE INVENTION

One object of the present disclosure is to provide a device which allows for sterile vascular access, e.g. placing a central venous catheter (CVC) on a patient, which removes or ameliorates at least some of the drawbacks of conventional methods.

Another object of the present disclosure is to provide a device which allows for sterile vascular access, e.g. placing a central venous catheter (CVC) on a patient, to be initiated, interrupted, and resumed again without the need to repeat all of the surgical preparations. This can promote time saving, increased ICU operational flexibility and reduced risk of infections for the patient.

Another object of the present disclosure is to provide a device which allows for sterile vascular access, e.g. placing a central venous catheter (CVC) on a patient, which may be handled by a single user.

The above objects as well as other objects that will become apparent to the skilled person in the light of the present disclosure are achieved by the various aspects of the invention as set out herein.

According to a first aspect of the disclosure, there is provided a sterile vascular access device for placing a central venous catheter (CVC) on a human subject, said device comprising:
a flexible enclosure formed of a flexible, transparent, pathogen impermeable medical grade material, said flexible enclosure having a base portion and a top portion, said base portion and said top portion together defining an internal space of the flexible enclosure,
said base portion having an adhesive bottom surface adapted to be reversibly
attached to the skin of the subject and a top surface connected to the top portion, and
said top portion comprising a tubular portion having a proximal end which is connected to the top surface of the base portion and a distal end which is sealed.

In use, the sterile vascular access device provides a local sterile environment during vascular access such as placement of a central venous catheter. The device facilitates rapid sterile placement of central venous catheters, by protecting the needle and catheter insertion site, needles, syringes, guidewires, and catheters, and optionally also local anesthetics from contamination by the external non-sterile environment.

The adhesive bottom surface of the base portion of the flexible enclosure is reversibly attached to the clean skin of the subject, the base portion and the top portion of the flexible enclosure together forming a sterile internal space above the insertion site.

The flexible enclosure is formed at least partially of a flexible and transparent medical grade material, preferably a plastic film. The flexible and transparent medical grade material is impermeable to pathogens, such as viruses and bacteria. The top portion of the flexible enclosure may allow items inside the flexible enclosure to be manipulated by fingers or hands of a user directly via the flexible walls of the flexible enclosure without compromising the sterility inside the flexible enclosure. The top portion of the flexible enclosure may further comprise one or more finger or hand shaped cavities in the walls of the flexible enclosure allowing fingers or hands of a user to reach into the inside the flexible enclosure without compromising the integrity and sterility inside of the flexible enclosure.

The inventive sterile vascular access device is particularly useful as it allows for sterile preparation and application of the vascular access device to be performed by a single user. Thus, using the inventive sterile vascular access device, all steps necessary for the preparation and application of the vascular access device can in principle be performed by a single health care provider. For example, preparation and application of the vascular access device may be performed by one person. Placement of the central venous catheter may be conveniently performed at a later time by the same person or by another person.

The inventive sterile vascular access device can be used for all types of central venous access (for example internal jugular vein, subclavian vein, axillary vein, femoral vein), arterial access (for example femoral artery, axillary artery, subclavian artery, radial artery, ulnar artery) and peripheral venous access. It is especially useful where space is limited, e.g. at the patient's neck.

The top portion of the flexible enclosure comprises a tubular portion having a proximal end which is connected to the top surface of the base portion and a distal end which is sealed. In some embodiments, the top portion of the flexible enclosure is comprised only of the tubular portion connected directly to the base portion. In other words, in some embodiments the proximal end of the tubular portion is connected directly to the top surface of said base portion. However, in some embodiments the tubular portion forms a part of the top portion. In a preferred embodiment, the top portion further comprises an intermediate bag portion, wherein the tubular portion is connected to the base portion via the intermediate bag portion.

The tubular shape of the tubular portion of the flexible enclosure provides several advantages for a device for placing a CVC on a human subject. For example, it allows the sterile supplies to be kept out of the way when the device is attached to the skin of the patient. It also allows for the flexible enclosure to be made with a small base portion. Both of these advantages are important for the vascular access device to be useful for placing a CVC on a human subject, where space is limited and the shape of the body can make attachment and sealing of the base portion to the skin of the subject difficult.

The tubular portion of the flexible enclosure preferably has an oblong shape having a length which is significantly greater than its width or diameter. The tubular portion may have different cross-sectional shapes, including but not limited to circular, oval, square or rectangular.

The length and width of the tubular portion may be suitably selected depending on the CVC supplies to be housed and handled within the tubular portion.

In some embodiments, the tubular portion has a length from the proximal end to the distal end in the range of 10-50 cm, preferably in the range of 15-45 cm.

In some embodiments, the tubular portion has a width in the range of 1-10 cm, preferably in the range of 2-8 cm, more preferably in the range of 2-6 cm.

In some embodiments, the length-to-width ratio of the tubular portion is greater than 2, preferably greater than 3, and more preferably greater than 4.

The length and width may refer to the length and width of the tubular portion in a deflated or inflated state, preferably in an inflated state.

As mentioned, in some embodiments the tubular portion forms a part of the top portion. In a preferred embodiment, the top portion further comprises an intermediate bag portion, and said tubular portion is connected to said base portion via the intermediate bag portion.

The intermediate bag portion preferably has a larger width than the tubular portion. Having an such an intermediate bag portion having a larger width arranged between the tubular portion and the base portion may improve visibility and maneuverability of the CVC supplies inside of the flexible enclosure during use. In some embodiments, the tubular portion extends in a direction non-parallel to, preferably transverse to, the extension of the intermediate bag portion from the base portion. Non-parallel or transverse extension of the tubular portion is advantageous both during attachment to the skin of the patient, and during the vascular access procedure, as it allows the CVC supplies to be kept out of the way.

In some embodiments, the top portion of the flexible enclosure comprises one or more cavities through which items inside the flexible enclosure may be manipulated by fingers or hands of a user. The one or more cavities may advantageously be placed on an intermediate bag portion having a larger width than the tubular portion.

Thus, in some embodiments, the intermediate bag portion comprises one or more cavities through which items inside the flexible enclosure may be manipulated by fingers or hands of a user. The cavities preferably comprise finger or hand shaped cavities in the walls of the flexible enclosure allowing fingers or hands of a user to reach into the inside the flexible enclosure without compromising the integrity of the flexible enclosure. The one or more cavities preferably include at least three finger shaped cavities or one hand shaped cavity. In some embodiments, one or more cavities are arranged on one side of the flexible enclosure and one or more cavities are arranged on another, preferably opposite, side of the flexible enclosure. The one or more cavities preferably include at least three finger shaped cavities or one hand shaped cavity on each of the opposing sides.

The base portion of the flexible enclosure has an adhesive bottom surface which is adapted to be reversibly attached to the skin of a subject. In a preferred embodiment, the bottom surface of the base portion is at least partially covered by a medical grade adhesive allowing it to be attached to the skin of the subject. In some embodiments, the entire base portion of the flexible enclosure is covered by the medical grade adhesive. In some embodiments, the base portion of the flexible enclosure is partially covered by the medical grade adhesive, e.g. in the form of a ring of adhesive surrounding an adhesive free area.

The medical grade adhesive on the bottom surface of the base portion may preferably be covered by a protective release liner which can be removed when the base portion is to be attached to the skin of a subject.

The size of the base portion should be small enough for the vascular access device to be useful for placing a central venous catheter (CVC) on a human subject, where space is limited, yet large enough to allow good adhesion and sealing against the skin of the subject where the shape of the body can make attachment and sealing of the base portion to the skin of the subject difficult. In some embodiments, the base portion adapted to be reversibly attached to the skin of the subject has a maximum width in the range of 5-20 cm, preferably in the range of 10-15 cm.

In some embodiments, the base portion is formed by a portion of the outside surface of the flexible enclosure at least partially covered by a medical grade adhesive.

In some embodiments, the base portion is in the form of a flange of flexible and transparent medical grade material extending in a radial direction, inwards and/or outwards, around a bottom periphery of the flexible enclosure.

In some embodiments, the base portion comprises a continuous film of flexible and transparent medical grade material. Such a base portion comprising a continuous film provides additional protection against contamination of the device prior to and during attachment to the skin of the patient. The skin of the patient can then be accessed from inside the device by either removing a portion of the continuous film or by inserting the needle into the skin directly through the continuous film.

In some embodiments, the base portion comprises at least one aperture through which the skin of the subject can be accessed. The aperture can be small, e.g. having a diameter of less than 1 cm, medium, e.g. having a diameter in the range of 1-6 cm, or large, e.g. having a diameter of more than 6 cm.

In some embodiments, the at least one aperture is arranged centrally in the base portion. Arranging the aperture centrally in the base portion provides for optimal adhesion and sealing around the aperture. However, in some cases it may be preferred to arrange the aperture closer to an edge of the base portion as this may facilitate ultrasound assisted localization of the relevant vessel. Thus, in some embodiments, the at least one aperture is disposed less than 3 cm, preferably less than 2 cm, more preferably less than 1 cm, or less than 0.5 cm, from an edge of the base portion.

An ultrasound device is often used to help locate the vein during placement of a CVC. An ultrasound device may be used from outside of the flexible enclosure, but this may require the presence of an ultrasound conductive medium inside and/or outside of the flexible enclosure to help conduct the ultrasound into the skin of the patient. Therefore, in some embodiments the flexible enclosure further comprises an ultrasound conductive medium.

The ultrasound conductive medium may also be applied on the patient's skin, or on the base portion, close to the needle and catheter insertion site. In some embodiments the base portion extends outside the top portion. In some of these embodiments ultrasound conductive medium that can be placed on top of the extending base portion. As an alternative, in some embodiments, the base portion further comprises an ultrasound conductive medium. The ultrasound conductive medium may for example be provided in a sealed pocket or vesicle in the base portion, close to the needle and catheter insertion site.

The sterile vascular access device may further comprise one or more adhesive tabs for securing the base portion to the skin of the subject. The adhesive tabs may for example be provided in the form of adhesive wings extending outwardly from the base portion. The adhesive tabs may be especially useful in an embodiment wherein an aperture in the base portion is arranged close to an edge of the base portion, resulting in a narrow adhesive region of potentially weaker adhesion and sealing between the aperture and the edge of the base portion.

In order to further facilitate handling of the sterile vascular access device when attached to a subject, the flexible enclosure may be inflated by introduction of a gas, typically medical grade oxygen or air. Inflating the flexible enclosure can improve visibility and maneuverability of the CVC supplies (e.g. hollow needle, syringe, guidewire, catheter, etc.) in the flexible enclosure. Thus, in some embodiments, the flexible enclosure is configured to be inflated after being attached to the skin of the subject. Introducing a positive pressure in the flexible enclosure also has the added advantage of preventing introduction of bacteria or other pathogens into the device if a minor leak should arise.

In some embodiments, the flexible enclosure comprises a gas inlet valve, and a gas outlet valve. In some embodiments, the device further comprises a gas filter capable of removing pathogens, such as viruses or bacteria from the incoming gas.

If the pressure in the flexible enclosure should become too high, this could in some stages of the CVC procedure lead to a risk of air embolisms, i.e. air being forced into the blood stream of the subject. In order to eliminate or reduce the risk of air embolisms, the flexible enclosure may be provided with a pressure relief valve, preventing the pressure in the flexible enclosure from becoming too high. A pressure relief valve is a type of safety valve used to control or limit the pressure in a system. In some embodiments, the flexible enclosure comprises a pressure relief valve. The central venous pressure of a human subject is typically in the range of 2-15 mmHg, and more commonly in the range of 8-12 mmHg. In order to prevent potentially problematic pressures, the pressure relief valve preferably has a pressure limit in the range of 1-5 mmHg, preferably in the range of 1-4 mmHg, and more preferably in the range of 2-4 mmHg.

The flexible enclosure of the inventive sterile vascular access device is advantageous since it can readily be produced from a single piece of the flexible and transparent medical grade material. Producing the flexible enclosure from a single piece of material minimizes the amount of joints required in the device, simultaneously reducing the risk of leakage and reducing the effort and cost of manufacturing the device.

In some embodiments, the base portion and the top portion are formed from a single piece of the flexible and transparent medical grade material.

Before use, supplies required for the vascular access procedure, i.e. for placing a CVC, must be introduced into the vascular access device. Examples of supplies include sterile hollow needles, syringes, guide wires, dilators, catheters, local anesthetics, fluids and adhesive film dressing. The supplies required for placing a CVC are often provided in the form of a CVC kit. The CVC kit typically comprises at least a hollow needle, a syringe, a guidewire and a catheter.

In preferred embodiments, the sterile vascular access device further comprises a CVC kit disposed in the tubular portion.

As the same type of CVC supplies are used in a vast majority of clinical cases, the inventive sterile vascular access device may advantageously be manufactured with a CVC kit already disposed in the flexible enclosure. The supplies of the CVC kit are preferably arranged in the flexible enclosure in a manner which allows the supplies to be readily used once the device has been attached to the skin of the subject, and optionally inflated. For example, the supplies of the CVC kit may preferably be placed in the tubular portion of the flexible enclosure and arranged to be accessed and used in the proper order.

In some embodiments, the device further comprises a holder for a CVC kit arranged in the tubular portion.

To allow the supplies of the CVC kit to be readily accessed and used in the proper order, the device may further comprise a holder for a CVC kit. The holder may for example comprise a structure having different slots configured to hold the different supplies. The holder may be fixed to the inside of the flexible enclosure or arranged loosely inside the flexible enclosure.

Although the sterile vascular access device may advantageously be manufactured with a CVC kit already disposed in the flexible enclosure, it may in some cases be preferred to allow the user to select the CVC supplies to be used. In order to allow the user to insert the selected CVC supplies into the flexible enclosure, the flexible enclosure may in some embodiments be provided with a hermetically sealable access port. The hermetically sealable access port may preferably be arranged at the distal end of the tubular portion, such that selected CVC supplies can be inserted directly into the tubular portion. Thus, in some embodiments, the distal end of the tubular portion is sealed by a hermetically sealable access port. The hermetically sealable access port may be provided in many different forms as known in the art and as realized by the skilled person. In some embodiments, the hermetically sealable access port comprises a hermetically sealable screw cap. A screw cap has been found to be a useful solution for the hermetically sealable access port since it provides good sealing capability with relatively low force.

In some embodiments, the sterile vascular access device comprises an anti-infective compound to further reduce the risks of infection. The anti-infective compound may be present in or coated on the flexible and transparent medical grade material or the medical grade adhesive or both. In a preferred embodiment an anti-infective compound is present in the medical grade adhesive to further increase the sterility during skin penetration of hollow needles and vascular access catheters. The anti-infective compound may for example be selected from the group consisting of hydrogen peroxide, povidone-iodine, cetylpyridinium chloride, cetylpyridinium bromide, 2-phenoxy ethanol, triclosan, polyhexanide, ethyl alcohol, isopropanol, bacitracin, gramicidin, fusidinic acid, polymyxine B, neomycin, gentamycin, erythromycin, chloramphenicol, lincosamide, minocycline, rifampin, doxycycline, or a gyrase inhibitor, or a combination thereof.

The inventive sterile vascular access device is advantageous in that sterile preparation and application of the vascular access device can be performed by a single user.

The inventive vascular access device is sterile. In order to maintain sterility of the device it is preferably provided in a sterile packaging container. Sterile packaging containers for medical supplies are well known in the art. The sterile packaging container typically comprises a tray and a cover. The cover can be removed from the tray to access the vascular access device placed on the tray.

According to a second aspect of the disclosure, there is provided a sterile vascular access device as defined above with reference to the first aspect, packaged in a sterile packaging container.

According to a third aspect of the disclosure, there is provided the use of a sterile vascular access device as defined above with reference to the first aspect for placing a central venous catheter (CVC) on a human subject.

According to a fourth aspect of the disclosure, there is provided a method placing a central venous catheter (CVC) on a human subject, comprising:
a) attaching a sterile vascular access device as defined above with reference to the first aspect to the disinfected skin of the subject over a vascular access site, said device further comprising a CVC kit disposed in the tubular portion, and
b) placing a CVC on the subject using the CVC kit.

The sterile vascular access device used in the second, third, and fourth aspect may be further defined as described above with reference to the first aspect.

### BRIEF DESCRIPTION OF THE DRAWINGS

Referring now to the drawings, which are exemplary embodiments, and wherein:
FIG. 1a is a schematic view of an embodiment of the sterile vascular access device;
FIG. 1b and 1c are schematic views of embodiments of the sterile vascular access device;
FIG. 2a is a schematic top view of an embodiment of the sterile vascular access device;
FIG. 2b is a schematic side view of an embodiment of the sterile vascular access device;
FIG. 3 is a schematic view of an embodiment of the sterile vascular access device;
FIG. 4 is a schematic view of an embodiment of the sterile vascular access device arranged on a patient during use.

### DETAILED DESCRIPTION OF PREFERRED EMBODIMENTS

Embodiments of the invention will now be described more in detail with reference to the drawings.

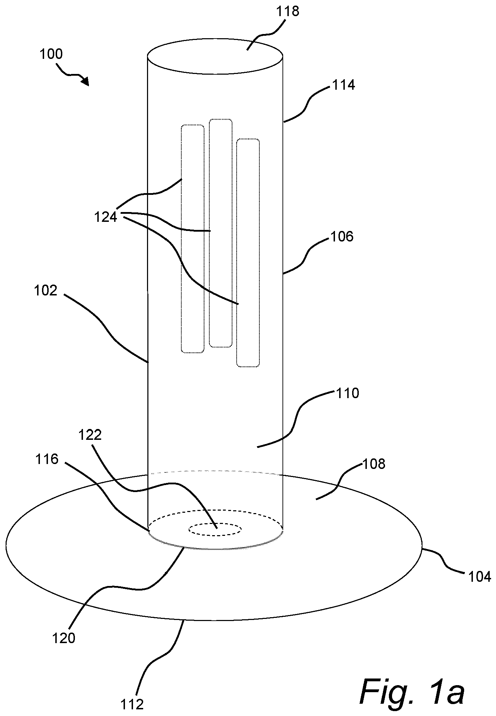

The basic constructional and functional features of an embodiment of the sterile vascular access device are illustrated in FIG. 1a. With reference to FIG. 1a, the disposable sterile vascular access device 100 for placing a central venous catheter (CVC) on a human subject comprises a flexible enclosure 102 having a base portion 104 and a top portion 106. The top portion 106 is connected to a top surface 108 of the base portion and the top portion 106 and the base portion together define an internal space 110 of the flexible enclosure.

The base portion 104 and top portion 106 of the flexible enclosure 102 is formed of a flexible and transparent medical grade material, specifically a plastic film. The flexible and transparent medical grade material is impermeable to bacteria and other pathogens. Transparency of the flexible enclosure is important in order for the CVC procedure to be carried out efficiently. The flexible enclosure is preferably made of polyolefin, such as polyethylene (PE), polypropylene (PP) or ethylene-vinyl acetate (EVA), but may also be made of various other medical grade plastics, including polyvinyl chloride (PVC), nylon, polyethylene terephthalate (PET), polyimide (PA), polycarbonate (PC), acrylonitrile butadiene (ABS), polyetheretherketone (PEEK) and polyurethane (PU). Particularly preferred materials for use as the flexible and transparent medical grade material of the device include polyethylene and copolymers thereof, such as ethylene-vinyl acetate. Ethylene-vinyl acetate is especially preferred, since in addition to providing a combination of good flexibility and mechanical strength, the transparency of ethylene-vinyl acetate films has been found to increase upon heating, such as the heating used when forming the film into a flexible enclosure. This characteristic of the ethylene-vinyl acetate films allows for thicker and more robust films to be used, while still maintaining high transparency. The thickness of the material is selected for a suitable combination of flexibility and mechanical strength. The top portion of the flexible enclosure is soft and flexible enough to allow items inside the flexible enclosure to be manipulated by the fingers or hands of a user directly via the flexible walls of the flexible enclosure. It has been found that the thickness of the material may preferably be in the range of 50-200 µm.

The flexible enclosure 102 may for example be manufactured by thermoforming, vacuum forming or pressure forming of a plastic sheet or film. The flexible enclosure may preferably be formed from a single piece of the flexible and transparent medical grade material. However, it is also possible to form different portions of the enclosure from different pieces of the same or different material(s) and then joining the different pieces, e.g. by plastic welding or gluing.

The base portion 104 has an adhesive bottom surface 112 adapted to be reversibly attached to the skin of a subject. The bottom surface 112 of the base portion 104 is covered by a medical grade adhesive. The medical grade adhesive may be suitably selected among medical grade adhesives, preferably pressure sensitive adhesives, known in the art for attaching e.g. adhesive bandages or electrodes to skin. The medical grade adhesive may for example include a rubber, an acrylate or a silicone formulation. The adhesive is commonly an acrylate, e.g. a methacrylate or an epoxy diacrylate. The medical grade adhesive may optionally further comprise an anti-infective compound to further reduce the risks of infection. The medical grade adhesive may be covered by a release liner (not shown in FIG. 1a), e.g. a low adhesive paper or plastic-based film sheet applied during the manufacturing process to prevent the adhesive bottom surface from prematurely adhering. The release liner protects the sterility and the adhesiveness of the medical grade adhesive before use. The release liner may optionally be provided with a tab, said tab facilitating removal of the release liner without touching the adhesive bottom surface.

The base portion 104, has a maximum width in the range of 5-20 cm, preferably in the range of 10-15 cm.

The top portion 106 is formed of a tubular portion 114 having a proximal end 116 which is connected to the top surface 108 of the base portion 104 and a distal end 118 which is sealed.

The tubular portion 114 has a length from the proximal end to the distal end in the range of 10-50 cm, preferably in the range of 15-45 cm.

The tubular portion 114 has a width in the range of 1-10 cm, preferably in the range of 2-8 cm, more preferably in the range of 2-6 cm.

The length and width refer to the length and width of the tubular portion in an inflated state.

In some embodiments, the base portion is in the form of a flange of flexible and transparent medical grade material extending in a radial direction (inwards and/or outwards) around the bottom periphery of the top portion. In the embodiment of FIG. 1a, the base portion 104 may be seen as a flange of the flexible and transparent medical grade material extending in a radial direction inwards and outwards from the bottom periphery 120 of the top portion 106.

The base portion 104 has an aperture 122 through which the skin of the subject can be accessed. The aperture is arranged centrally in the base portion. Arranging the aperture centrally in the base portion provides for optimal adhesion and sealing around the aperture 122. The aperture 122 can be small, e.g. having a diameter of less than 1 cm, medium, e.g. having a diameter in the range of 1-6 cm, or large, e.g. having a diameter of more than 6 cm.

In alternative embodiment, the base portion may comprise a continuous film of flexible and transparent medical grade material, i.e. without an aperture. Such a base portion comprising a continuous film provides additional protection against contamination of the device prior to and during attachment to the skin of the patient. The skin of the patient can then be accessed from inside the device by either removing a portion of the continuous film or by inserting the needle into the skin directly through the film of the base portion.

For use, the base portion of the flexible enclosure is attached to the clean skin of the subject using the medical grade adhesive, the top portion of the flexible enclosure forming a sterile space above the insertion site. The device facilitates rapid sterile placement of a CVC, by protecting the needle and catheter insertion site, needles, guidewires and catheters from contamination by the external non-sterile environment.

Before use, sterile supplies required for the vascular access procedure, i.e. for placing a CVC, must be introduced into the vascular access device 100. Examples of supplies include sterile hollow needles, syringes, guide wires, dilators, catheters, local anesthetics, fluids and adhesive film dressing. The supplies required for placing a CVC are often provided in the form of a CVC kit. The CVC kit typically comprises at least a hollow needle, a syringe, a guidewire and a catheter.

As the same type of CVC supplies are used in a vast majority of clinical cases, the inventive sterile vascular access device may advantageously be manufactured with a CVC kit already disposed in the flexible enclosure. The supplies of the CVC kit are preferably arranged in the flexible enclosure in a manner which allows the supplies to be readily used once the device has been attached to the skin of the subject, and optionally inflated. For example, the supplies of the CVC kit may preferably be placed in the tubular portion of the flexible enclosure and arranged to be accessed and used in the proper order.

To allow the supplies of the CVC kit to be readily accessed and used in the proper order, the device may further comprise a holder for a CVC kit. The holder (not shown) may for example comprise a structure having different slots configured to hold the different supplies. The holder may be fixed to the inside of the flexible enclosure or arranged loosely inside the flexible enclosure.

In the embodiment of FIG. 1a, the sterile vascular access device comprises a CVC kit 124 disposed in the tubular portion.

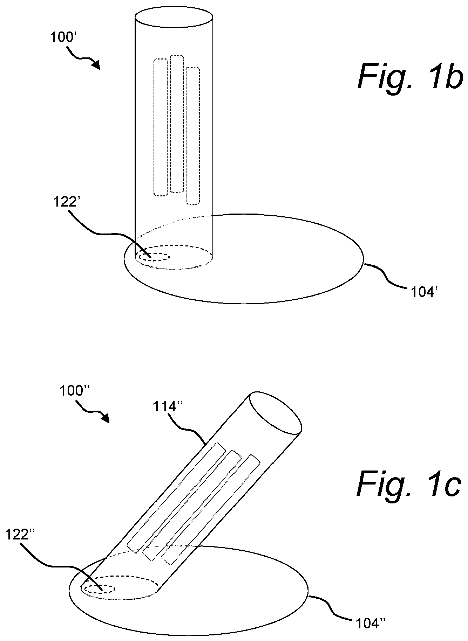

In some cases, it may be preferred to arrange the aperture closer to an edge of the base portion as this may facilitate ultrasound assisted localization of the relevant vessels. In the embodiment of FIG. 1b the aperture 122' is disposed less than 3 cm, preferably less than 2 cm, more preferably less than 1 cm, from an edge of the base portion 104'.

The tubular portion may be provided in different shapes and configurations, e.g. to save space or to offer a better working angle. In the embodiment of FIG. 1c the tubular portion 114" is arranged at an angle against the base portion 104" and the aperture 122" is disposed less than 3 cm, preferably less than 2 cm, more preferably less than 1 cm, from an edge of the base portion 104".

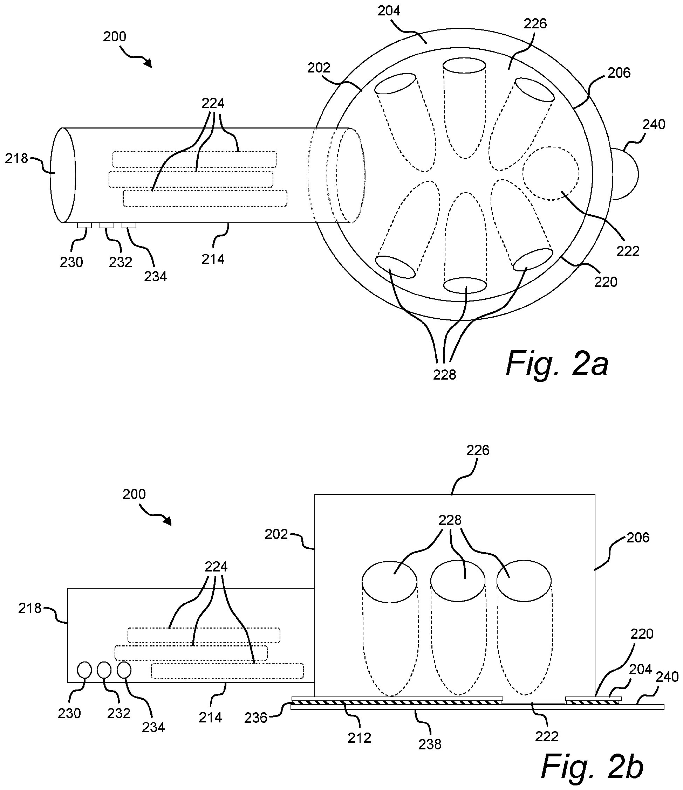

FIG. 2a and 2b show an embodiment of the sterile vascular access device 200. The device 200 comprises a flexible enclosure 202 having a base portion 204 and a top portion 206. The top portion 206 comprises a tubular portion 214 and an intermediate bag portion 226, wherein the tubular portion 214 is connected to said base portion via the intermediate bag portion 226.

The intermediate bag portion 226 has a larger width than the tubular portion 214. The larger width of the intermediate bag portion improves visibility and maneuverability of the CVC supplies inside of the flexible enclosure during use.

The base portion 204 may be seen as a flange of the flexible and transparent medical grade material extending in a radial direction inwards and outwards from the bottom periphery 220 of the top portion 206.

The base portion 204 has an aperture 222 through which the skin of the subject can be accessed.

In the embodiment of FIG. 2a and 2b, the sterile vascular access device 200 comprises a CVC kit 224 disposed in the tubular portion 214.

The intermediate bag portion further comprises six finger shaped cavities 228 in the walls of the enclosure, allowing fingers or hands of a user to reach into the inside the intermediate bag portion 226 without compromising the integrity or sterility of the inside of the flexible enclosure. The finger shaped cavities may for example be arranged to receive thumb, index finger and middle finger of each hand of the user. Three of the finger shaped cavities are arranged on one side of the intermediate bag portion and the remaining tree finger shaped cavities are arranged on the opposite side of the intermediate bag portion. The finger shaped cavities 228 allow the user to access the needle and catheter insertion site, and to handle the CVC kit including necessary supplies, such as needles, syringes, guidewires and catheters, inside of the flexible enclosure 202. Although the finger shaped cavities 228 in the figures are arranged in a row, it is also contemplated that the cavities may be arranged in a more ergonomic pattern, e.g. in a triangular pattern, allowing the fingers of the user to work better together. In some embodiments, finger shaped cavities arranged on one side of the intermediate bag portion are displaced relative to finger shaped cavities arranged on the opposite side of the intermediate bag portion, allowing the fingers of both hands of the user to work better together.

The base portion 204 has an adhesive bottom surface 212 adapted to be reversibly attached to the skin of a subject. The bottom surface 212 of the base portion 204 adapted to be attached to the skin of a subject is covered by a medical grade adhesive 236. The medical grade adhesive 236 is covered by a release liner 238, e.g. a low adhesive paper or plastic-based film sheet applied during the manufacturing process to prevent the adhesive bottom surface from prematurely adhering. The release liner protects the sterility and the adhesiveness of the medical grade adhesive before use. The release liner may optionally be provided with a tab 240, said tab facilitating removal of the release liner without touching the adhesive bottom surface.

In order to further facilitate handling of the sterile vascular access device when attached to a subject, the flexible enclosure may be provided with means for inflating the enclosure by introduction of a gas, typically medical grade oxygen or air. Inflating the bag can further improve visibility and maneuverability of the CVC supplies (e.g. needle, guidewire, catheter, etc.) in the flexible enclosure. Introducing a positive pressure in the flexible enclosure also has the added advantage of preventing introduction of bacteria or other pathogens into the device if a minor leak should arise.

To allow for inflation of the flexible enclosure, the vascular access device comprises a gas inlet valve 230, and a gas outlet valve 232. The gas inlet valve and gas outlet valve are arranged near the distal end 218 of the tubular portion 214.

Gas (for example oxygen, air, NO₂ or antiseptic gas) from a suitable gas source can be fed through the vascular access device to keep the flexible enclosure inflated. The level of inflation of the flexible enclosure can be regulated by controlling the gas flow through the gas inlet valve and gas outlet valve respectively. The gas flow may typically be in the range of 0.1-10 l/min. A gas flow at a higher end of the range may be used for inflating the enclosure and a gas flow closer to the lower end of the range may then be used for maintaining the degree of inflation

In order to eliminate or reduce the risk of air embolisms, the flexible enclosure is provided with a pressure relief valve, preventing the pressure in the flexible enclosure from becoming too high. A pressure relief valve is a type of safety valve used to control or limit the pressure in a system. In some embodiments, the flexible enclosure comprises a pressure relief valve. The central venous pressure of a human subject is typically in the range of 2-15 mmHg, and more commonly in the range of 8-12 mmHg. In order to prevent potentially problematic pressures, the pressure relief valve preferably has a pressure limit in the range of 1-5 mmHg, preferably in the range of 1-4 mmHg, and more preferably in the range of 2-4 mmHg. The pressure relief valve 234 of the embodiment in FIG 2a and 2b has a pressure limit of 2.8 mmHg.

The parts of the vascular access device, e.g. the base portion and the intermediate bag portion, can be manufactured using conventional plastic manufacturing methods well known to a person skilled in the art. In some embodiments, the flexible enclosure, including the base portion, the tubular portion and the intermediate bag portion, are formed as a single continuous part. In other embodiments, the different portions are formed individually and then joined by conventional techniques, such as plastic welding or gluing. A preferred joining technique is high frequency welding (HFwelding).

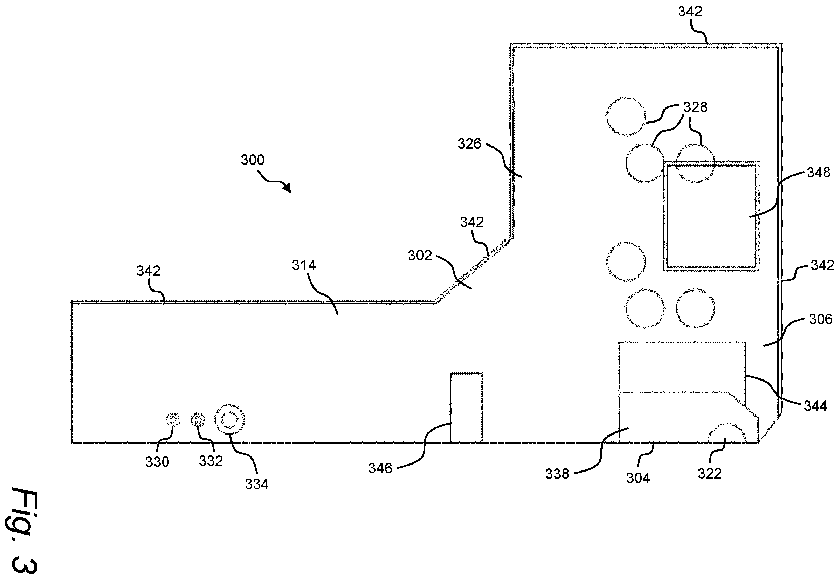

FIG. 3 shows an embodiment of the vascular access device 300 wherein the flexible enclosure 302 is formed from a single piece of flexible and transparent medical grade material. The flexible enclosure is formed from a piece of plastic film which has been folded in half. The folded halves have then been welded together along a weld line 342 to form the flexible enclosure. The flexible enclosure 302 has a base portion 304 and a top portion 306. The base portion 304 is formed by a portion of the outside surface of the flexible enclosure which has been covered by a medical grade adhesive. The medical grade adhesive is covered and protected by a protective release liner 338 which can be removed when the base portion is to be attached to the skin of a subject. The top portion 306 comprises a tubular portion 314 and an intermediate bag portion 326, wherein the tubular portion 314 is connected to said base portion via the intermediate bag portion 326.

The intermediate bag portion 326 has a larger width than the tubular portion 314. The larger width of the intermediate bag portion improves visibility and maneuverability of the CVC supplies inside of the flexible enclosure during use.

The base portion 304 and top portion 306 of the flexible enclosure 302 is formed of a flexible and transparent medical grade material, specifically a plastic film. The flexible and transparent medical grade material is impermeable to bacteria and other pathogens. Transparency of the flexible enclosure is important in order for the CVC procedure to be carried out efficiently. The top portion 306 may optionally include a window 348 formed of a plastic film with higher transparency.

The medical grade adhesive and a protective release liner, as well as other details, including an aperture 322 in the base portion, adhesive tabs 344 for securing the base portion to the skin of the subject, finger cavities 328 in the intermediate bag portion, a gas inlet 330, a gas outlet 332, a pressure relief valve 334, a holder 346 for a CVC kit, and a window 348 of high transparency material, may advantageously be added to the piece of plastic film before it is folded and welded together. In some embodiments, the device further comprises a gas filter (not shown) at the gas inlet capable of removing pathogens, such as viruses or bacteria from the incoming gas. The gas filter may for example be a standard 0.1-0.4 µm sterile filter.

The inventive vascular access device is sterile. Sterilization of the device may be performed using different methods known to the person skilled in the art, depending on the construction and materials of the device. A preferred sterilization method is gamma irradiation.

In order to maintain sterility of the device it is preferably provided in a sterile packaging container. Sterile packaging containers for medical supplies are well known in the art. The sterile packaging container typically comprises a tray and a cover. The cover can be removed from the tray to access the vascular access device placed on the tray.

This arrangement is especially useful since it allows sterile preparation and application of the vascular access device to be performed by a single user. Thus, using the inventive sterile vascular access device kit, all steps necessary for the preparation and application of the vascular access device can in principle be performed by a single health care provider.

For the vascular access procedure, the skin is initially cleaned with for example 2% chlorhexidine in 70% alcohol.

Taking care not to contaminate the adhesive bottom surface, the release liner is removed from the base portion of the sterile vascular access device, and the adhesive bottom surface of the base portion of the device containing the sterile supplies in the tubular portion is attached to the disinfected skin of the subject over the vascular access site, for example at the internal jugular vein. Hereby, the sterility of the skin and the skin facing surface of the vascular access device is secured.

The sterile vascular access device is connected to a gas supply and inflated to improve visibility and maneuverability. The level of inflation of the flexible enclosure can be regulated by controlling the gas flow through the gas inlet valve and gas outlet valve respectively.

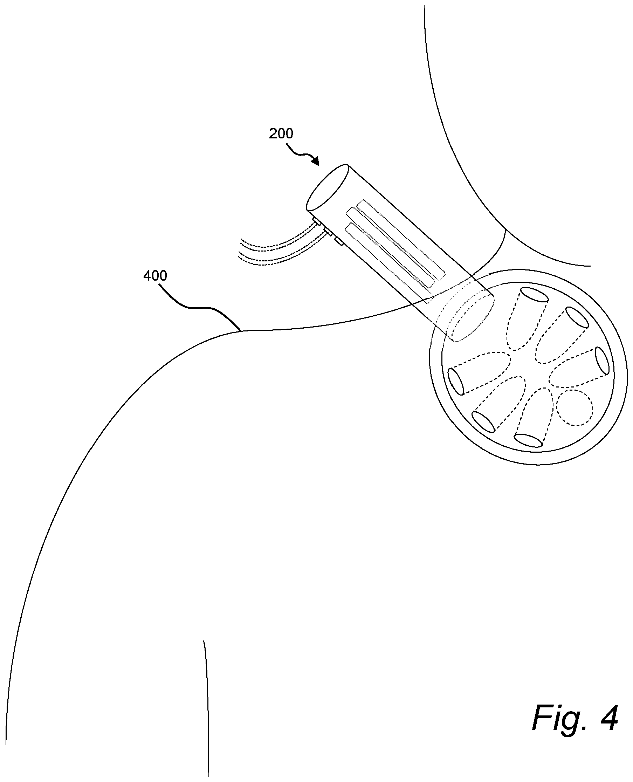

FIG. 4 shows the sterile vascular access device 200 of FIG 2a and 2b arranged on a patient 400 during use. The base portion of the flexible enclosure is attached to the skin of the patient such that the flexible enclosure forms a sterile space above the needle and catheter insertion site. The sterile supplies required for placing the CVC are positioned in the tubular portion. The tubular portion reduces the amount of space required for the device at the patient's neck and conveniently keeps supplies not in immediate use out of the way. The flexible enclosure is inflated by gas (for example oxygen, air, NO₂ or antiseptic gas) from a suitable gas source (not shown) fed through the flexible enclosure.

The vascular access procedure is performed by for example ultrasound localization of the relevant vessel, insertion of a hollow needle into the central vein, guidewire insertion through the hollow needle into the central vein, removing the hollow needle, tissue dilation, catheter over the wire inserting the central venous catheter in the central vein, and removing the guidewire. With the central venous catheter in place, the vascular access device is detached from the skin of the patient and removed. The central venous catheter may then be sutured (and/or otherwise fixated) in place and a transparent sterile dressing placed over part of the central venous catheter. In addition, the central venous catheter can be filled with antiseptic solution in order to increase sterility. The vascular access device can be used in combination with antibiotic prophylaxis to further reduce infection rates.

Although the vascular access device described herein is particularly useful for central venous catheter placement, it should be understood that it may also be used with similar principles and with the same or similar equipment, or with other equipment, for central venous access (for example internal jugular vein, subclavian vein, axillary vein or femoral vein), arterial access (for example femoral artery, axillary artery, radial artery, ulnar artery or brachial artery), or peripheral venous access (for example hand vein, fore arm vein, basilic vein, cephalic vein, brachial vein or leg vein). In addition, the vascular access device may be used with similar principles for implantation of devices (for example pacemakers, implantable cardioverter-defibrillators or cardiac implantable electric devices) or tunneled central catchers (for example Hickmann, Groshong). Furthermore, the vascular access device may be used for open surgery and/or laparoscopic surgery. In addition, the vascular access device may be used for regional or neuraxial anesthesia. In each application the vascular access device may be equipped with relevant supplies or equipment needed for that specific procedure. Other possible uses for the vascular access device include abdominal aortic aneurysm repair, limb amputation; appendix surgery, AV shunt for dialysis, bile duct, liver, or pancreatic surgery, breast surgery, cardiac surgery, coronary bypass with chest and donor incisions, coronary bypass graft, carotid endarterectomy, extracorporeal membrane oxygenation (ECMO), gallbladder surgery, colon surgery, craniotomy, caesarean section, spinal fusion, open reduction of fracture, gastric surgery, herniorrhaphy, hip prosthesis, heart transplant, abdominal hysterectomy, knee prosthesis, kidney transplant, laminectomy, liver transplant, neck surgery, kidney surgery, ovarian surgery, pacemaker surgery, prostate surgery, peripheral vascular bypass surgery, rectal surgery, small bowel surgery, spleen surgery, thoracic surgery, thyroid and/or parathyroid surgery, vaginal hysterectomy, ventricular shunt, and exploratory laparotomy.

The vascular access device may be provided together with equipment kits customizable for any procedure requiring sterility.

The vascular access device may also be used in changing of vascular access dressings.

The vascular access device may also be used in the veterinary field, prehospital field, battlefield, unsanitized places, intensive care units, emergency rooms, hospital wards, dental care, lab procedures, human testing, and/or pharmaceutical testing, manufacturing, research, or delivery.

While the invention has been described herein with reference to various exemplary embodiments, it will be understood by those skilled in the art that various changes may be made and equivalents may be substituted for elements thereof without departing from the scope of the invention. In addition, many modifications may be made to adapt a particular situation or feature to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

## Claims

1. A sterile vascular access device for placing a central venous catheter (CVC) on a human subject, said device comprising:
a flexible enclosure formed of a flexible, transparent, pathogen impermeable medical grade material, said flexible enclosure having a base portion and a top portion, said base portion and said top portion together defining an internal space of the flexible enclosure,
said base portion having an adhesive bottom surface adapted to be reversibly attached to the skin of the subject and a top surface connected to the top portion, and
said top portion comprising a tubular portion having a proximal end which is connected to the top surface of the base portion and a distal end which is sealed.

2. The sterile vascular access device according to any one of the preceding claims, wherein the proximal end of the tubular portion is connected directly to the top surface of said base portion.

3. The sterile vascular access device according to any one of the preceding claims, wherein the tubular portion has a length from the proximal end to the distal end in the range of 10-50 cm, preferably in the range of 15-45 cm.

4. The sterile vascular access device according to any one of the preceding claims, wherein the tubular portion has a width in the range of 1-10 cm, preferably in the range of 2-8 cm, more preferably in the range of 2-6 cm.

5. The sterile vascular access device according to any one of the preceding claims, wherein the top portion further comprises an intermediate bag portion, and wherein said tubular portion is connected to said base portion via the intermediate bag portion.

6. The sterile vascular access device according to claim 5, wherein the intermediate bag portion has a larger width than the tubular portion.

7. The sterile vascular access device according any one of claims 5-6, wherein the tubular portion extends in a direction non-parallel to, preferably transverse to, the extension of the intermediate bag portion from the base portion.

8. The sterile vascular access device according to any one of claims 5-7,
wherein the intermediate bag portion comprises one or more cavities through which items inside the flexible enclosure may be manipulated by fingers or hands of a user.

9. The sterile vascular access device according to any one of the preceding claims, wherein the base portion adapted to be reversibly attached to the skin of the subject has a maximum width in the range of 5-20 cm, preferably in the range of 10-15 cm.

10. The sterile vascular access device according to any one of the preceding claims, wherein the base portion comprises at least one aperture through which the skin of the subject can be accessed.

11. The sterile vascular access device according to claim 10, wherein said at least one aperture is disposed less than 3 cm, preferably less than 2 cm, more preferably less than 1 cm, from an edge of the base portion.

12. The sterile vascular access device according to any one of the preceding claims, wherein the base portion further comprises an ultrasound conductive medium.

13. The sterile vascular access device according to any one of the preceding claims, wherein the device further comprises one or more adhesive tabs for securing the base portion to the skin of the subject.

14. The sterile vascular access device according to any one of the preceding claims, wherein the flexible enclosure is configured to be inflated after being attached to the skin of the subject.

15. The sterile vascular access device according to any one of the preceding claims, wherein the flexible enclosure comprises a gas inlet valve, and a gas outlet valve.

16. The sterile vascular access device according to any one of the preceding claims, wherein the flexible enclosure comprises a pressure relief valve.

17. The sterile vascular access device according to any one of the preceding claims, wherein said base portion and said top portion are formed from a single piece of the flexible and transparent medical grade material.

18. The sterile vascular access device according to any one of the preceding claims, wherein the device further comprises a holder for a CVC kit arranged in the tubular portion.

19. The sterile vascular access device according to any one of the preceding claims, wherein the device further comprises a CVC kit disposed in the tubular portion.

20. The sterile vascular access device according to any one of the preceding claims, wherein the distal end is sealed by a hermetically sealable access port.

21. The sterile vascular access device according to any one of the preceding claims, wherein sterile preparation and application of the vascular access device can be performed by a single user.

22. A sterile vascular access device kit, comprising:
a sterile vascular access device according to any one of claims 1-21 packaged in a sterile packaging container.

23. Use of a sterile vascular access device according to any one of claims 1-21 for placing a central venous catheter (CVC) on a human subject.
